# FASCICULE DE BREVET EUROPEEN

(11) **EP 2 445 429 B1**
(45) Date de publication et mention de la délivrance du brevet: **23.11.2016**
(21) Numéro de dépôt: 10734158.8
(22) Date de dépôt: 11.06.2010
(51) Int. Cl.: A61B 17/72

(54) **CLOU D'ALLONGEMENT POUR OS LONG OU ANALOGUE**
EXPANSIONSNAGEL FÜR LANGE KNOCHEN UND DERGLEICHEN
ELONGATION NAIL FOR LONG BONE OR SIMILAR

(30) Priorité: 24.06.2009 FR 0903051
(43) Date de publication de la demande: 02.05.2012
(73) Titulaire: Guichet, Jean-Marc, 13008 Marseille (FR)
(72) Inventeur: Guichet, Jean-Marc, 13008 Marseille (FR)
(74) Mandataire: Flavenot, Bernard
(86) Numéro de dépôt international: PCT/FR2010/000429
(87) Numéro de publication internationale: WO 2010/149868

(56) Documents cités:
- EP-A1- 0 346 247
- WO-A1-95/24870
- WO-A1-96/15377
- US-A- 5 704 939

## Description

### Domaine d'activité

L'invention se rattache au secteur technique des éléments de prothèses utilisés notamment en chirurgie orthopédique, et plus particulièrement ceux qui sont connus sous le terme de clous d'allongement pour os longs ou analogues.

On sait que, suite à des malformations ou anomalies congénitales, ou bien suite à des fractures, à une perte de substance osseuse, ou autre, certains os, tels que l'humérus, le fémur, le tibia, etc. sont trop courts, créant ainsi une asymétrie ou une hypométrie.

Dans le cas des membres inférieurs, pour une asymétrie faible, inférieure à trois centimètres, on a recours à l'adjonction, du côté du membre le plus court, d'une semelle ou d'une chaussure orthopédique.

### Techniques chirurgicales et historique

Pour une asymétrie plus importante ou une hypométrie, on dispose, pour obtenir un allongement du membre le plus court, de solutions chirurgicales utilisant actuellement deux types de moyens à base respectivement de fixateurs externes ou de fixateurs internes.

Dans la technique à base de fixateurs externes, on utilise un appareillage externe dont certaines parties (par exemple des broches métalliques) traversent les tissus mous pour atteindre l'os auxquels elles sont solidarisées. L'appareillage est donc accessible à l'extérieur du membre et l'avantage de cette technique est de permettre aux Praticiens d'agir sur ledit appareillage à des périodes déterminées pour provoquer de manière progressive un certain degré d'allongement de l'os au niveau généralement de la diaphyse de l'os, après section de celle-ci. Cependant, ces fixateurs externes présentent notamment les inconvénients suivants : ils sont encombrants et peu esthétiques, les patients sur lesquels ils sont implantés les supportent parfois difficilement, et ils entraînent des risques d'infection non négligeables.

La technique à base de fixateurs internes consiste à procéder d'une manière interne, c'est-à-dire de façon qu'aucun élément n'apparaisse à l'extérieur du membre. Pour cela, on utilise généralement des clous dits centro-médullaires ou des plaques d'ostéosynthèse convenablement fixées à l'os. L'avantage de cette solution par rapport à la précédente réside dans le fait qu'aucun élément n'apparaît à l'extérieur du membre. En revanche, il n'est possible d'obtenir un allongement de l'os que grâce à des interventions chirurgicales itératives consistant, pour chacune d'elles, à obtenir un gain fixe, mais limité du fait de la limite élastique et plastique des tissus mous à l'étirement. De plus, en cas d'allongement important non progressif, le comblement de l'écart dimensionnel est aléatoire et nécessite des adjuvants à l'ostéosynthèse, par exemple des greffes osseuses, qui diminuent les propriétés mécaniques et physiologiques de l'os allongé.

### Technique du clou d'allongement

Pour palier ces inconvénients mentionnés ci-dessus, le Demandeur a réalisé un clou d'allongement initial (CAI) qui a fait l'objet d'une protection par brevets, notamment le EP 0 346 247, voir préambule de la revendication3 1. Cette réalisation a pour but de créer un système d'allongement qui combine les avantages d'un allongement progressif modulable à souhait à l'aide des fixateurs externes, et les avantages des fixateurs internes, sans pour autant présenter les inconvénients de ces deux systèmes d'allongement.

D'autres clous d'allongement ont été réalisés.

Par exemple, il en existe un qui fonctionne par rotations effectuées lors de manoeuvres non contrôlables en toute sécurité. Le patient peut se sur-allonger en très peu de temps, ce qui peut conduire à des complications neurologiques ou des non-consolidations osseuses.

Il en existe un autre, comme celui décrit dans le EP-A-1477123, qui ne fonctionne pas par rotation, mais avec deux tubes s'éloignant l'un de l'autre en translation au moyen d'une pièce fixée au bassin qui, de ce fait, empêche une mobilité de la hanche.

Un autre, dit "clou électronique", est décrit dans le US-A-6245075. Il comporte une commande électrique, ce qui induit des problèmes de sécurité. Une absence de mise en charge pendant l'allongement en a limité l'intérêt clinique.

On peut aussi citer le clou d'allongement décrit dans le document US-A-5 704 939.

Ces systèmes d'allongement selon l'art antérieur n'ont pas diminué les qualités du clou d'allongement initial (CAI) mentionné ci-dessus ni son intérêt, car ils présentent certains défauts, notamment un manque d'appui complet pendant l'allongement et un fonctionnement aléatoire avec des échecs.

### Principe du fonctionnement du clou d'allongement initial (CAI)

Le clou d'allongement initial (CAI) selon le document cité ci-dessus est remarquable en ce qu'il est agencé pour être fixé en vue de rendre solidaires au moins deux parties d'un même os long sectionné en deux parties, et constitué d'éléments aptes à lui conférer un allongement progressif résultant d'une rotation partielle appliquée par manoeuvre externe sur le membre concerné, et/ou acquis automatiquement lors de mouvements physiologiques du membre, par exemple la marche, et d'éléments pour son maintien dans la position d'allongement après chaque rotation appliquée sur le membre et le retour en position neutre des parties osseuses des segments de membre concernés.

Ce clou comprend essentiellement un fourreau à l'intérieur duquel est monté, avec capacité de déplacement en translation, un tube assujetti à des moyens aptes à provoquer le déplacement relatif du tube et du fourreau sous l'effet de mouvements de rotation appliqués au membre dans un sens, pour correspondre à un allongement de l'os, et, dans l'autre sens de rotation, à réaliser le blocage en translation du tube et du fourreau. La translation est obtenue au moyen de cliquets à dents.

La présente invention a pour but, notamment, d'améliorer structurellement ce clou CAI et son fonctionnement, d'optimiser les résultats cliniques obtenus, de diminuer les cliquetages qui sont parfois difficiles voire douloureux, d'améliorer l'appui limité pendant l'allongement, de réduire les difficultés de positionnement des deux tubes, de réduire les difficultés pour son extraction s'il est entièrement enseveli dans l'os, et de lui permettre de coopérer avec un même outil ancillaire quelle que soit la taille de ce clou.

Plus précisément, la présente invention a pour objet un clou d'allongement pour os long ou analogue, selon la revendication 1.

D'autres caractéristiques et avantages de la présente invention apparaîtront au cours de la description suivante donnée en regard des dessins annexés à titre illustratif mais nullement limitatif, dans lesquels :
La figure 1 est une demi-coupe longitudinale d'un mode de réalisation du clou d'allongement selon l'invention, et
La figure 2 est une vue schématique en coupe d'un détail de la réalisation selon la figure 1, encerclé en C sur la figure 1.

La présente invention concerne un clou d'allongement pour os long ou analogue, du même type que celui défini dans le document antérieur EP-A-0346247 cité dans le préambule à la présente description.

Le clou d'allongement selon l'invention comporte un premier tube creux 10 d'un diamètre intérieur d'une première valeur, un second tube 20 d'un diamètre extérieur d'une seconde valeur au plus égale à la première valeur, ce second tube, creux ou non, mais de préférence plein et cylindrique de révolution pour une question de rigidité, étant monté coulissant et rotatif dans le premier tube 10 de façon qu'une première 21 de ses deux extrémités émerge d'une première 11 des deux extrémités du premier tube 10, et des moyens 30 pour coupler les premier et second tubes 10, 20 de façon que des oscillations rotatives successives des deux tubes l'un par rapport à l'autre autour de leur axe longitudinal 100 entraînent une translation longitudinale pas-à-pas des deux tubes l'un par rapport à l'autre.

Un mode de réalisation de ces moyens 30 pour coupler les deux tubes dans le but défini ci-avant sera donné ci-après, conforme à la structure décrite dans le EP-A-0346247.

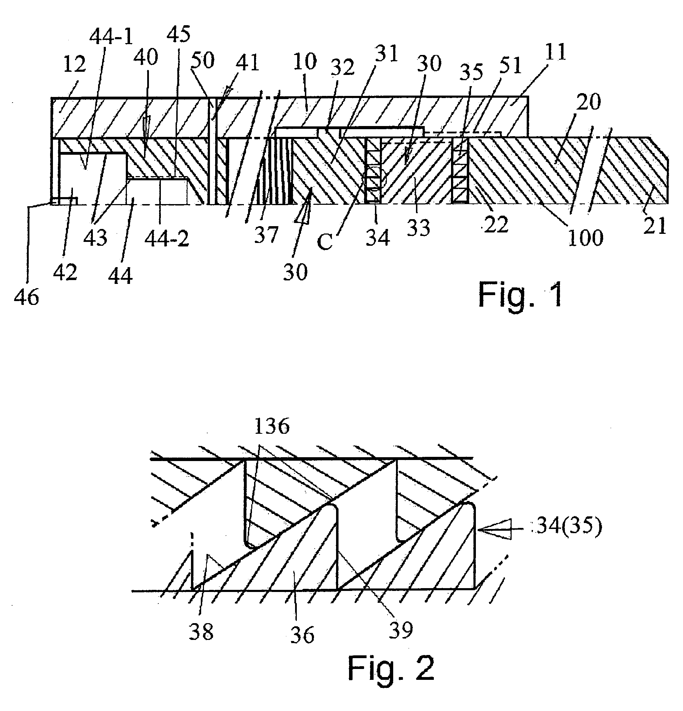

Selon l'invention comme illustré sur la figure 1, la seconde extrémité 12 du premier tube 10 est ouverte et le clou comporte un embout 40 monté, de préférence complètement enfiché, dans cette seconde extrémité ouverte 12 du premier tube 10, et des moyens 41 pour bloquer cet embout 40 par rapport au premier tube.

Ces moyens 41 pour bloquer l'embout comportent au moins une goupille 50 traversant à la fois au moins une partie de la paroi du premier tube 10 et au moins une partie de la paroi de l'embout 40, comme illustré sur la figure 1.

Selon une caractéristique essentielle de l'invention, l'embout 40 comporte des moyens de coopération 42 avec une patte d'un outil ancillaire pour la pose du clou (non représentée) de façon que cette patte soit apte à être introduite dans la seconde extrémité 12 du premier tube 10 pour coopérer avec l'embout 40, par exemple par enfichage.

De façon avantageuse, ces moyens de coopération 42 sont constitués par l'une 43 des deux parties d'un emboîtement mâle-femelle.

De façon préférentielle, cette partie 43 d'emboîtement mâle-femelle est la partie femelle 44, de façon qu'il n'y ait aucun élément oblong formant saillie sur la paroi latérale du tube 10, comme un ergot ou analogue, ni une cavité, dans le but d'éviter, après l'implantation du premier tube dans l'os, la formation d'une ossification autour de l'ergot ou dans la cavité, qui empêcherait l'extraction du clou.

En revanche, cet embout 40 totalement enfiché dans l'extrémité 12 du premier tube 10, va favoriser l'extraction du clou lorsque la valeur souhaitée pour l'allongement de l'os aura été obtenue, essentiellement grâce à la partie femelle 44 coopérant avec une partie mâle de la patte de l'ancillaire.

En outre, pour une sécurité d'implantation du clou d'allongement, il est avantageux que cette partie femelle 44 comporte, comme illustré sur la figure 1, au moins deux portions femelles consécutives 44-1, 44-2 selon l'axe longitudinal 100 défini précédemment.

Ces deux portions femelles consécutives 44-1, 44-2 ont des sections transversales différentes, la section transversale de la portion femelle 44-1 la plus proche de la seconde extrémité 12 du premier tube 10 étant supérieure à celle de l'autre portion femelle 44-2.

Elles sont donc avantageusement constituées de deux cavités de forme sensiblement cylindrique de révolution, la paroi de la cavité la plus proche 44-1 de la seconde extrémité 12 du premier tube 10 étant sensiblement lisse pour constituer un guide, la paroi de l'autre cavité 44-2 portant un filetage de fixation 45.

Le clou d'allongement comporte en outre des moyens 46 pour indexer la patte de l'ancillaire par rapport au premier tube creux 10 et/ou à l'embout 40, puisque cet embout et le premier tube 10 sont fixes l'un par rapport à l'autre, de façon à faciliter le positionnement de la patte de cet ancillaire et du clou par rapport à l'os à allonger. Ces moyens d'indexation peuvent être constitués, comme illustré sur la figure 1, par une encoche réalisée dans la paroi intérieure du premier tube 10 et/ou dans l'embout 40.

Quant aux moyens 30 pour coupler les premier et second tubes 10, 20 de façon que des oscillations rotatives des ces deux tubes l'un par rapport à l'autre entraînent une translation longitudinale pas-à-pas des deux tubes l'un par rapport à l'autre, ils comportent avantageusement, comme illustré sur la figure 1, au moins un doigt 31 monté coulissant dans le premier tube 10 entre l'embout 40 et la seconde extrémité 22 du second tube 20, des moyens 32 pour empêcher ce doigt 31 de subir une rotation sur lui-même dans le premier tube 10, une douille filetée 33 montée rotative en vissage dans un filetage complémentaire 51 réalisé sur la paroi intérieure du premier tube 10, cette douille filetée étant disposée entre le doigt 31 et la seconde extrémité 22 du second tube 20, deux couples de cliquets 34, 35 complémentaires à dents 36 sensiblement de forme générale en dents de scie, ces deux couples de cliquets 34, 35 étant montés respectivement en coopération entre le doigt 30 et la douille filetée 33, et entre la douille filetée 33 et la seconde extrémité 22 du second tube 20, chaque couple de cliquets ayant des sens de rotation opposés, et des moyens de ressort de compensation 37 pour absorber l'éloignement relatif des trois éléments : doigt, douille filetée et second tube, quand les dents 36 d'un même couple 34, 35 passent l'une sur l'autre. Ces moyens de ressort 37, comme un ressort en spirale ou analogue, sont calibrés pour éviter des commandes par cliquetages intempestifs et permettre de réguler le gain d'allongement en le limitant quasi-exclusivement aux manoeuvres volontaires du patient.

Selon une réalisation préférentielle, pour faciliter le passage des dents les unes sur les autres, comme illustré sur la figure 2 qui représente à plus grande échelle la partie encerclée référencée C sur la figure 1, chaque dent 36 comporte un flanc oblique 38 et un flanc raide 39 se rejoignant à un sommet 136 qui présente une forme fortement arrondie.

De façon avantageuse, et qui a donné toute satisfaction au Demandeur, au moins l'un des couples de cliquets, de préférence les deux couples, comporte deux paires d'environ vingt-quatre dents. Cette dernière caractéristique améliore la qualité des manoeuvres cliniques rotatoires et augmente les caractéristiques mécaniques du clou selon l'invention en autorisant en outre une diminution globale des dimensions en calibre des éléments respectivement référencés 10, 20, 30 et 40.

Le fonctionnement du clou d'allongement selon l'invention est le même que celui décrit dans le document antérieur EP-A-0 346 247 précité. Il ne sera donc pas plus amplement développé ici.

En revanche, il est utile de mentionner ici les avantages du clou selon la présente invention, qui sont des conséquences des caractéristiques structurelles de perfectionnement par rapport à celles du clou d'allongement initial (CAI).

A savoir, notamment :
- une facilitation du positionnement du clou et de son extraction avec l'embout 40 à double échappement et filetage interne, même si le clou est enseveli dans l'os et la néo-ossification,
- une suppression des cavités en surface du tube 10, évitant ainsi une ossification gênant l'extraction du clou,
- un changement de morphologie des cliquets augmentant la sécurité et autorisant des calibres inférieurs de clou et une facilitation du cliquetage,
- un contrôle accru des manoeuvres de cliquetage avec diminution potentielle des phénomènes douloureux.

## Revendications

1. Clou d'allongement pour os long ou analogue, comportant :
• un premier tube creux (10) d'un diamètre intérieur d'une première valeur,
• un second tube (20) d'un diamètre extérieur d'une seconde valeur au plus égale à ladite première valeur, ledit second tube étant monté coulissant et rotatif dans ledit premier tube (10) de façon qu'une première (21) des deux extrémités du second tube émerge d'une première (11) des deux extrémités du premier tube (10), et
• des moyens (30) pour coupler les deux dits tubes (10, 20) de façon que des oscillations rotatives successives des deux tubes l'un par rapport à l'autre autour de leur axe longitudinal (100) entraînent une translation longitudinale pas-à-pas des deux tubes l'un par rapport à l'autre, la seconde extrémité (12) du premier tube (10) étant ouverte et ledit clou comportant en outre un embout (40) monté complètement enfiché dans cette seconde extrémité (12) du premier tube et des moyens (41) pour bloquer ledit embout (40) par rapport au dit premier tube, **caractérisé par le fait que** ledit embout comporte des moyens de coopération (42) avec une patte d'un outil ancillaire pour la pose du clou, ladite patte étant apte à être introduite dans la seconde extrémité (12) du premier tube (10) pour coopérer avec ledit embout (40).

2. Clou selon la revendication 1, **caractérisé par le fait que** lesdits moyens de coopération (42) avec une patte d'un outil ancillaire sont constitués par l'une (43) des deux parties d'un emboîtement mâle-femelle.

3. Clou selon la revendication 2, **caractérisé par le fait que** ladite partie (43) d'emboîtement mâle-femelle est la partie femelle (44).

4. Clou selon la revendication 3, **caractérisé par le fait que** ladite partie femelle (44) comporte deux portions femelles consécutives (44-1, 44-2) selon ledit axe longitudinal (100).

5. Clou selon la revendication 4, **caractérisé par le fait que** les deux portions femelles (44-1, 44-2) ont des sections transversales différentes, la section transversale de la portion femelle (44-1) la plus proche de la seconde extrémité (12) du premier tube (10) étant supérieure à celle de l'autre portion femelle (44-2).

6. Clou selon la revendication 5, **caractérisé par le fait que** les deux portions femelles consécutives (44-1, 44-2) sont constituées de deux cavités de forme sensiblement cylindrique de révolution, la paroi de la cavité la plus proche (44-1) de la seconde extrémité (12) du premier tube (10) étant sensiblement lisse pour constituer un guide, la paroi de l'autre cavité (44-2) portant un filetage de fixation (45).

7. Clou selon l'une des revendications précédentes, **caractérisé par le fait que** les moyens (41) pour bloquer ledit embout (40) par rapport au premier tube (10) comportent au moins une goupille (50) traversant à la fois au moins une partie de la paroi du premier tube (10) et au moins une partie de la paroi du dit embout (40).

8. Clou selon l'une des revendications précédentes, **caractérisé par le fait que** les moyens (30) pour coupler les deux tubes (10, 20) de façon que des oscillations rotatives des deux tubes (10, 20) l'un par rapport à l'autre entraînent une translation longitudinale pas-à-pas des deux tubes l'un par rapport à l'autre, comportent au moins :
• un doigt (31) monté coulissant dans le premier tube (10) entre ledit embout (40) et la seconde extrémité (22) du second tube (20),
• des moyens (32) pour empêcher ledit doigt (31) de subir une rotation sur lui-même dans ledit premier tube (10),
• une douille filetée (33) montée rotative en vissage dans un filetage complémentaire (51) réalisé sur la paroi intérieure du premier tube (10), ladite douille filetée étant disposée entre ledit doigt (31) et la seconde extrémité (22) du second tube (20),
• deux couples de cliquets (34, 35) complémentaires à dents (36) sensiblement en forme de dents de scie, ces deux couples de cliquets (34, 35) étant montés respectivement en coopération entre ledit doigt (30) et ladite douille filetée (33), et entre ladite douille filetée et la seconde extrémité (22) du second tube (20), chaque couple de cliquets ayant des sens de rotation opposés, et
• des moyens de ressort de compensation (37) pour absorber l'éloignement relatif des trois éléments : doigt, douille filetée et second tube, quand les dents (36) d'un même couple (34, 35) passent l'une sur l'autre.

9. Clou selon la revendication 7, **caractérisé par le fait que** chaque dent (36) comporte un flanc oblique (38) et un flanc raide (39) se rejoignant à un sommet (136) de forme fortement arrondie pour faciliter le passage des dents les unes sur les autres.

10. Clou selon l'une des revendications 8 et 9, **caractérisé par le fait qu'**au moins l'un des couples de cliquets comporte deux paires d'environ vingt-quatre dents.

11. Clou selon l'une des revendications précédentes, **caractérisé par le fait qu'**il comporte des moyens (46) pour indexer la patte de l'ancillaire par rapport au premier tube creux (10) et/ou à l'embout (40), ces moyens étant constitués par une encoche réalisée dans la paroi intérieure du premier tube (10) et/ou dans l'embout (40).

## Patentansprüche

1. Verlängerungsnagel für Langknochen oder dergleichen, umfassend:
- ein erstes Hohlrohr (10) mit einem Innendurchmesser eines ersten Wertes,
- ein zweites Rohr (20) mit einem Außendurchmesser eines zweiten Wertes, der höchstens gleich dem ersten Wert ist, wobei das zweite Rohr in das erste Rohr (10) verschiebbar und drehbar eingesetzt ist, derart, dass ein erstes (21) der zwei Enden des zweiten Rohrs aus einem ersten (11) der zwei Enden des ersten Rohrs (10) herausragt, und
- Mittel (30) zum Koppeln der zwei Rohre (10, 20) in der Weise, dass aufeinander folgende rotatorische Oszillationen der zwei Rohre relativ zueinander um ihre Längsachse (100) eine schrittweise Längsverschiebung der zwei Rohre relativ zueinander zur Folge haben, wobei das zweite Ende (12) des ersten Rohrs (10) offen ist und der Nagel außerdem einen Ansatz (40), der so angebracht ist, dass er vollständig in dieses zweite Ende (12) des ersten Rohrs eingesteckt ist, und Mittel (41), um den Ansatz (40) in Bezug auf das erste Rohr festzusetzen, aufweist, **dadurch gekennzeichnet, dass** der Ansatz Mittel (42) für die Zusammenwirkung mit einer Lasche eines Hilfswerkzeugs für das Einbringen des Nagels aufweist, wobei die Lasche in das zweite Ende (12) des ersten Rohrs (10) einführbar ist, um mit dem Ansatz (40) zusammenzuwirken.

2. Nagel nach Anspruch 1, **dadurch gekennzeichnet, dass** die Mittel (42) für die Zusammenwirkung mit einer Lasche eines Hilfswerkzeugs als eines (43) von zwei Teilen einer Stecker-Buchsen-Verbindung ausgebildet sind.

3. Nagel nach Anspruch 2, **dadurch gekennzeichnet, dass** der Teil (43) der Stecker-Buchsen-Verbindung der Buchsenteil (44) ist.

4. Nagel nach Anspruch 3, **dadurch gekennzeichnet, dass** der Buchsenteil (44) zwei entlang der Längsachse (100) aufeinander folgende Buchsenabschnitte (44-1, 44-2) aufweist.

5. Nagel nach Anspruch 4, **dadurch gekennzeichnet, dass** die zwei Buchsenabschnitte (44-1, 44-2) unterschiedliche Querschnitte haben, wobei der Querschnitt des Buchsenabschnitts (44-1), der sich am nächsten bei dem zweiten Ende (12) des ersten Rohrs (10) befindet, größer als jener des anderen Buchsenabschnitts (44-2) ist.

6. Nagel nach Anspruch 5, **dadurch gekennzeichnet, dass** die zwei aufeinander folgenden Buchsenabschnitte (44-1, 44-2) als zwei Hohlräume mit im Wesentlichen drehzylindrischer Form ausgebildet sind, wobei die Wand des Hohlraums, der sich am nächsten (44-1) bei dem zweiten Ende (12) des ersten Rohrs (10) befindet, im Wesentlichen glatt ist, um eine Führung zu bilden, während die Wand des anderen Hohlraums (44-2) ein Befestigungsgewinde (45) trägt.

7. Nagel nach einem der vorhergehenden Ansprüche, **dadurch gekennzeichnet, dass** die Mittel (41) zum Festsetzen des Ansatzes (40) in Bezug auf das erste Rohr (10) wenigstens einen Stift (50) aufweisen, der sowohl wenigstens durch einen Teil der Wand des ersten Rohrs (10) als auch wenigstens durch einen Teil der Wand des Ansatzes (40) verläuft.

8. Nagel nach einem der vorhergehenden Ansprüche, **dadurch gekennzeichnet, dass** die Mittel (30) zum Koppeln der zwei Rohre (10, 20) in der Weise, dass rotatorische Oszillationen der zwei Rohre (10, 20) relativ zueinander eine schrittweise Längsverschiebung der zwei Rohre relativ zueinander zur Folge haben, wenigstens umfassen:
- einen Zapfen (31), der in dem ersten Rohr (10) zwischen dem Ansatz (40) und dem zweiten Ende (22) des zweiten Rohrs (20) verschiebbar montiert ist,
- Mittel (32), um zu verhindern, dass der Zapfen (31) in dem ersten Rohr (10) einer Drehung um sich selbst unterliegt,
- eine Gewindehülse (33), die in einem komplementären Gewinde (51), das an der Innenwand des ersten Rohrs (10) ausgebildet ist, drehverschraubt montiert ist, wobei die Gewindehülse zwischen dem Zapfen (31) und dem zweiten Ende (22) des zweiten Rohrs (20) angeordnet ist,
- zwei komplementäre Klinkenpaare (34, 35) mit Zähnen (36), die im Wesentlichen Sägezahnform haben, wobei diese zwei Klinkenpaare (34, 35) jeweils zwischen dem Zapfen (30) und der Gewindehülse (33) und zwischen der Gewindehülse und dem zweiten Ende (22) des zweiten Rohrs (20) zusammenwirkend angebracht sind, wobei jedes Klinkenpaar entgegengesetzte Drehrichtungen hat, und
- Ausgleichsfedermittel (37), um die relative Verrückung der drei Elemente: Zapfen, Gewindehülse und zweites Rohr, aufzunehmen, wenn sich die Zähne (36) desselben Paars (34, 35) aneinander vorbei bewegen.

9. Nagel nach Anspruch 7, **dadurch gekennzeichnet, dass** jeder Zahn (36) eine schräge Flanke (38) und eine steile Flanke (39) aufweist, die sich an einem Scheitelpunkt (136) mit stark abgerundeter Form treffen, um die Vorbeibewegung der Zähne aneinander zu erleichtern.

10. Nagel nach einem der Ansprüche 8 und 9, **dadurch gekennzeichnet, dass** wenigstens eines der Klinkenpaare zwei Paare mit etwa vierundzwanzig Zähnen aufweist.

11. Nagel nach einem der vorhergehenden Ansprüche, **dadurch gekennzeichnet, dass** er Mittel (46) umfasst, um die Lasche des Hilfsgeräts in Bezug auf das erste Hohlrohr (10) und/oder den Ansatz (40) zu indexieren, wobei diese Mittel als eine Kerbe ausgebildet sind, die in der Innenwand des ersten Rohrs (10) und/oder in dem Ansatz (40) ausgebildet ist.

## Claims

1. Elongation nail for long bone or the like, comprising:
• a first hollow tube (10) with an inner diameter of a first value;
• a second tube (20) with an outer diameter of a second value at most equal to said first value, said second tube being mounted to slide and rotate in said first tube (10) so that a first (21) of both ends of the second tube emerges from a first (11) of both ends of the first tube (10); and
• means (30) for coupling both said two tubes (10, 20) so that successive alternating rotations of the two tubes in relation to one another about their longitudinal axis (100) result in a longitudinal step-by-step translation of the two tubes in relation to one another, the second end (12) of the first tube (10) being open and said nail further comprising a insert (40) mounted completely inserted in the second end (12) of the first tube and means (41) for fixing said insert (40) with respect to said first tube, **characterized in that** said insert comprises means for cooperation (42) with one lug of an ancillary tool for setting of the nail, said lug being adapted to be inserted into the second end (12) of the first tube (10) to cooperate with said insert (40).

2. Nail according to claim 1, **characterized in that** said means for cooperation (42) with one lug of an ancillary tool consist of one (43) of the two parts of a male-female joint.

3. Nail according to claim 2, **characterized in that** said part (43) of the male-female joint is the female part (44).

4. Nail according to claim 3, **characterized in that** said female part (44) has two consecutive female portions (44-1, 44-2) along said longitudinal axis (100).

5. Nail according to claim 4, **characterized in that** the two female portions (44-1, 44-2) have different cross sections, the cross section of the female portion (44-1) closest to the second end (12) of the first tube (10) being greater than that of the other female portion (44-2).

6. Nail according to claim 5, **characterized in that** the two consecutive female portions (44-1, 44-2) consist of two substantially cylindrical chambers, the wall of the cavity nearest (44-1) the second end (12) of the first tube (10) being substantially smooth to form a guide, the wall of the other cavity (44-2) having a mounting thread (45).

7. Nail according to any one of the previous claims, **characterized in that** the means (41) for fixing said insert (40) in relation to the first tube (10) comprise at least one pin (50) through both at least part the wall of the first tube (10) and at least part of the wall of said insert (40).

8. Nail according to any one of the previous claims, **characterized in that** the means (30) for coupling the two tubes (10, 20) so that the alternating rotations of the two tubes (10, 20) with respect to one another cause a step-by-step longitudinal translation of the two tubes in relation to one another, comprising at least:
• a finger (31) slidably mounted in the first tube (10) between said insert (40) and the second end (22) of the second tube (20);
• means (32) for preventing said finger (31) from being rotated on itself in said first tube (10);
• a threaded socket (33) rotatably mounted in a complementary thread screw (51) formed on the inner wall of the first tube (10), said threaded socket being disposed between said finger (31) and the second end (22) of the second tube (20);
• two pairs of complementary ratchet bosses (34, 35) with teeth (36) having a substantially sawtooth form, these two pairs of ratchet bosses (34, 35) being mounted respectively in cooperation between said finger (30) and said threaded sleeve (33), and between said threaded sleeve and the second end (22) of second tube (20), each pair of ratchet bosses having opposite directions of rotation; and
• compensating spring means (37) for absorbing the relative separation of three elements: finger, threaded socket and second tube, when the teeth (36) of a given pair (34, 35) move past each other.

9. Nail according to claim 7, **characterized in that** each tooth (36) has an oblique slope (38) and a right slope (39) joining at a vertex (136) strongly rounded in shape to facilitate passage of each tooth on the other.

10. Nail according to claims 8 or 9, **characterized in that** at least one of the pairs of ratchet bosses are two pairs of about twenty-four teeth.

11. Nail according to any one of the previous claims, **characterized in that** it comprises means (46) to index the lug of an ancillary instrument from the first hollow tube (10) and/or the insert (40), these means being constituted by a notch formed in the inner wall of the first tube (10) and/or the insert (40).
